# EUROPEAN PATENT SPECIFICATION

(11) **EP 0 307 071 B1**
(45) Date of publication and mention of the grant of the patent: **14.05.1997**
(21) Application number: 88305760.6
(22) Date of filing: 24.06.1988
(51) Int. Cl.: C12N 1/14, C12N 9/42, C12N 5/00, C12N 15/00, A23L 1/211, A23L 1/015

(54) **Flavor and fragrance enhancing enzymes**
Geruchs- und Geschmacksverstärkende Enzyme
Enzymes pour fortifier la saveur et la fragance

(30) Priority: 24.06.1987 IL 82980
(43) Date of publication of application: 15.03.1989
(73) Proprietor: YISSUM RESEARCH DEVELOPMENT COMPANY OF THE HEBREW UNIVERSITY OF JERUSALEM, Jerusalem 91042 (IL)
(72) Inventor: Shoseyov, Oded, Rehovot (IL); Chet, Ilan, Nes Ziona (IL); Bravdo, Ben-Ami, Rehovot (IL); Ikan, Raphael, Jerusalem (IL)
(74) Representative: Sheard, Andrew Gregory

(56) References cited:
- DD-A- 253 640
- PATENT ABSTRACTS OF JAPAN, unexamined applications, C field, vol. 9, no. 52, March 6, 1985 THE PATENT OFFICE JAPANESE GOVERNMENT page 85 C 269
- CHEMICAL ABSTRACTS, vol. 100, no. 3, January 16, 1984, Columbus, Ohio, USA H. BLOHM: "Partial characteri-zation of the thermostable anthocyanin- -glycosidase fromAspergillus niger" page 237, left column, abstract no. 19 670y
- INDIAN JOURNAL OF BIOCHEMISTRY & BIOPHSICS, vol. 22, April 1985 (New Delhi, India) H.V. ADIKANE et al.: "Isolation and Properties of -Glucosidase from Aspergillus niger" pages 97-101
- CHEMICAL ABSTRACTS, vol. 91, no. 5, July 30, 1979, Columbus, Ohio, USA D. JEAN et al.: "Cultivation of a strain of Aspergillus niger that produces tannase (tannin acyl hydrolase). Appliction to gallotannin degradation" page 576, right column, abstract-no. 37 458k
- Columbus, Ohio, USA D. JEAN et al.: " Cultivation of a strain of Aspergillus niger that produces tannase (tannin acyl hydrolase ). Application to gallotannin degradation" page 576, right column

## Description

The present invention is directed toward enzymes which are capable of increasing the flavour or fragrance of plants, fruits, plant extracts, and fermentation products. The invention is also directed toward methods for preparing and using enzymes having such activities. The invention is also directed to *Aspergillus niger* strains, and a recombinant molecule capable of expressing such an enzyme.

The characteristic flavours, aromas and fragrances of plants (such as fruits, leaves, roots and flowers etc.) result from the synthesis and presence of compounds known as "monoterpene alcohols" (Aryan, A.P. *et. al. Amer. J. Enol.* **38**:182-188 (1987); Grossmann, M. *et. al., Deutsche Lebensmit. Rund.* **83**: 7-12 (1987)) which references are herein incorporated by reference) . Monoterpenes are found in plant tissue in two major forms, free (i.e. not bound to a sugar residue) odorous monoterpenes and odorless bound (i.e. bonded to a sugar) monoterpene glycosides. Both the bound and the free monoterpenes content increase during plant growth and throughout maturation. During the maturation of the plant, the odorous monoterpenes are converted into flavorless glucosyl derivatives (such as O-α-L-rhamnopyranosyl-β-D-glucopyranosides, and O-α-L-arabinofuranosyl-β-D-glycopyranosides). At harvest many plants therefore contain high concentrations of odorless monoterpene glycosides which may serve as an additional source for aroma if hydrolyzed. Since the above-discussed conversion results in a loss of flavor, it would be highly desirable to develop a process capable of reversing this conversion and thereby increasing the concentration of flavorants in plant materials.

Several approaches designed to hydrolyze the glucosyl derivatives and liberate monoterpenes have been investigated. Acid treatment, for example, has been found to be capable of achieving such hydrolysis, however, the treatment also results in an undesirable rearrangement of the monoterpenes (Williams, P.J. et al., J. Argric. Food Chem. 30:1219-1223 (1982)).

The discovery of glucosidases, which are enzymes capable of catalyzing the hydrolysis of glucosyl compounds, has led to investigations of the possible use of such enzymes to increase the concentration of monoterpenes in fruit juices (Aryan, A.P. et al. Amer. J. Enol. 38:182-188 (1987)).

In order for a glucosidase to be useful in hydrolyzing glucosyl derivatives of monoterpene, the enzyme must be capable of exhibiting substantial activity in the presence of a high concentration of sugars (up to approximately 1.5 M, such as is naturally present in fruit juices, or much higher concentrations present in juice concentrates). A suitable enzyme must also exhibit substantial activity as the low pH characteristic of natural fruit juice (pH 2-4). To be suitable in the treatment of fermented juices, the enzyme must be capable of exhibiting substantial activity in the presence of 3-15% ethanol. Additionally, the enzyme should have high resistance to SO₂.

### ENDO-β-GLUCOSIDASES

Endo-β-glucosidases have been found to be produced by a wide variety of plants and animals. Maguire, R.J. disclosed the identification of a cellobiose beta glucosidase from Trichoderma viride (Maguire, R.J. Can. J. Biochem. 55:19-26 (1977). This enzyme is reported to have a log rate-pH maxima at pH 5.2, and to be strongly inhibited by sugar. A glucosidase has been identified from Lipomyces starkeyi which has a low optimum pH, and a high optimum temperature. This enzyme is, however, an endo alpha-glucosidase, and not an endo-β-glucosidase (Kelly, C.T. et al., Appl. Microbiol. Biotechnol. 22:352-358 (1985)). Duff and co-workers identified endo-β-glucosidase enzymes from Trichoderma reesei and Aspergillus phoenicus which were more tolerant to glucose concentration; these enzymes were, however, temperature labile, and had a pH activity maxima of 4.0-4.75 (Duff, S.J.B. et al., Enzyme Microb. Technol. 8:305-308 (1986)). A similar heat labile endo-β-glucosidase was isolated from the dalmatian sage, Salvia officinalis (Brzunov, K. et al., Acta Pharm. Jugosl. 35:175-180 (1985)). An endo-β-glucosidase from honey has been disclosed which has a pH optima of 4.2, and a temperature optima of 60 C (Low, N.H. et al., J. Apiculture Res. 25:178-181 (1986)). An endo-β-glucosidase has been isolated from rose petals (Bugorskii, P.S. et al., Biokhimiya 44:1068-1073 (1079); Biochemistry (english translation of Biokhimiya) 44: 841-845 (1980)). Bayonove and co-workers isolated a endo-β-glucosidase from muscat grapes (Bayonove, C. et al., Bull. OIV 643:741-758 (1984); Cordonnier, R. et. al., Comptes Rendus Herbdomad. Seances Acad. Sci. 278:3387-3390 (1974)). This enzyme was found to have low juice enzyme activity. The unsuitability of this enzyme was attributed to the assumed presence of natural enzymatic inhibitors in the juice (Bayonove, C. et al., Bull. OIV 643:741-758 (1984)). Darrere, M. (Semana Vitivinicola 40:2209-2211 (1985)) discusses the use of a commercially available endo-β-glucosidase to break down alcohol-insoluble glucones in wine.

Recently, Aryan, A.P. et al. studied the enzymatic characteristics of a glucosidase from the muscat of Alexandria (Vitis vinifera), and compared these characteristics to those of commercially available pectolytic enzymes (Rohapect C and almond beta-glucosidase (emulsin) EC 3.2.1.21) in an effort to identify an enzyme which would be suitable for catalyzing the release of juice monoterpenes (Aryan, A.P. et al. Amer. J. Enol. 38:182-188 (1987)). The enzymes were found to be virtually inactive at the low pH of natural fruit juice, and were found to be strongly inhibited by sugar. The enzymes were, moreover, found to be incapable of hydrolyzing the glucosyl derivatives of certain of the most flavor-important monoterpenes. Aryan et al. concluded that none of the studied beta-glucosidase enzymes have any practical utility in the hydrolysis of an glucosyl derivative of a monoterpene in wine or fruit juice.

### ENDO-BETA-GLUCOSIDASES OF ASPERGILLUS AND NEUROSPORA

Endo-β-glucosidase have been isolated from strains of the mold, Aspergillus. Srivastava, S.K. et al. (Enzym. Microb. Technol. 6:508-512 (1984)) disclose an endo-β-glucosidase of Aspergillus wentii. The enzyme was found to be capable of functioning at high temperature, but exhibited decreased activity at pH of less than 5.5. An endo-β-glucosidase has been isolated from Aspergillus niger by Adikane and associates (Adikane, H.V. et al., Indian J. Biochem. Biophys. 22:97-101 (1985)). This enzyme was found to have a molecular weight of 170 kd, to have a pH optima of 4-5, and a temperature optima of 60 C. A similar enzyme has been isolated from Aspergillus niger by Park, K.H. et al. (J. Kor. Agric. Chem. Soc. 24:186-193 (1981)). This enzyme had maximal activity at a pH of 4.7 and at a temperature of 60 C. A strain of Neurospora crassa has been isolated which produces an endo-beta-glucosidase having pH optima of 4-5, and a temperature optima of 50-60 C (Macris, B.J. *et. al. Biotechnol. Lett.* **9:**661-664 (1987)).

Japanese patent application No. 58-63074 discloses that the bitterness of an aloe extract can be removed by treating the extract with a microorganism or an enzyme to hydrolyze a glycoside. The microorganism may be *Aspergillus niger* and the enzyme may be β glucosidase.

*Food Chemistry* **12:** 197-204 (1983) discloses that an anthocyanin-β-glycosidase (anthocyanase) from *Aspergillus niger* has been partially purified and shown to catalyse lysis of the β glycosidic bond of anthocyanins.

The above-discussed investigations have not led to the identification of an endo-beta-glucosidase which possesses the characteristics necessary for the efficient hydrolysis of the glucosyl derivatives of a monoterpene of wine and fruit juices. Thus, at present, no endo-beta-glucosidase has been identified which has the characteristics required for use in improving the flavour of wine or fruit juice.

According to the present invention there is provided *Aspergillus niger* Bl strain CMI CC 324626 or a mutant thereof.

Thus, the invention relates to novel mutant strains of *A. niger,* designated *"A. niger* Bl". It also relates to a method for producing such mutant strains. The invention further relates to flavour and fragrance enhancing enzymes which can be isolated from this novel mutant, and to functional derivatives of such enzymes.

According to the present invention, there is further provided a flavour or fragrance enhancing enzyme obtainable from *Aspergillus niger* Bl which is strain CMI CC 324626, or a functional derivative thereof, characterised in that the enzyme has endo-beta glucosidase activity, has an optimum of glucosidase activity at about pH 3.4, has enhanced glucosidase activity in a culture medium that has up to about 50% alcohol content and has glucosidase activity at 60°C.

Further features of the present invention are set out in the claims.

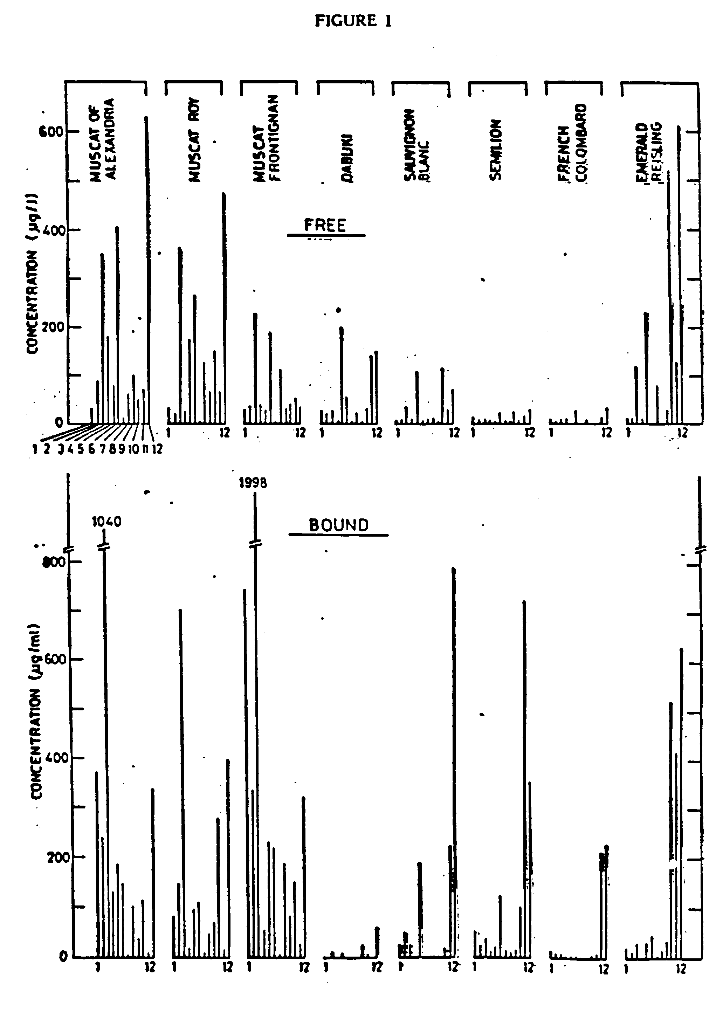

Figure 1 shows the concentrations of free and bound monoterpenes in various wines. The bound monoterpenes were assayed by isolating them from wines and incubating them with endo-β-glucosidase Bl, followed by extraction with Freon 11 and injection into a gas chromatograph.

The present invention derives, in part, from the isolation of a novel strain of *Aspergillus niger,* designated *"Aspergillus niger* Bl strain CMI CC 324626" which exhibits several enzymatic activities that are useful in enhancing the flavour or fragrance of plants (including flowers, fruit, leaves, roots, vegetable parts, etc.) plant extracts, or fermentation products. The term "fermentation product" is intended to refer to the material obtained through the fermentation of plant material. Examples of such fermentation products include wine, beer, etc.

Among the enzymatic activities identified in extracts of *A. niger* Bl strain CMI CC 324626 are an endo-beta-glucosidase, a tannase, and an anthocyanase activities. The endo-β-glucosidase enzyme is suitable for use in the hydrolysis of a glucosyl or glycosyl bond present in a glucosyl or glycosyl derivative of a monoterpene, to yield free monoterpenes. The tannase and anthocyanase activities are useful in decreasing the amount of tannins and anthocyanins in plants, juices, wines, etc. The present invention includes the endo-β-glucosidase enzyme in a form which is "substantially free of natural contaminants". An enzyme is said to be substantially free of natural contaminants if it has been produced or purified in a manner which has led to the loss, removal, or absence of compounds with which the enzyme is naturally found or associated. Examples of such compounds include the unfractionated lipids, carbohydrates, and proteins of the cytoplasm of an *Aspergillus niger* cell.

The process for obtaining *A. niger* Bl strain CMI CC 324626 or its equivalents comprises cultivating a conventional *A. niger* strain, such as *A. niger* Clone No. 5 on a medium of purified monoterpene glycosides as sole carbon source and other nutrients at a temperature of about 30°C and a pH of about 3.4 for a number of weeks, homogenation and further cultivation for a number of days, resulting in a massive mycelium which sporulates after a few days, and selecting from the spores a source of an *A. niger* strain having the desired characteristics. *A. niger* B1 strain CMI CC 324626 was isolated in this manner, and such isolation is reproducible by the man versed in the art.

*Aspergillus niger* Bl strain CMI CC 324626 can be mutagenized by means well known in the art in order to obtain mutant derivatives which are capable of producing either an enhanced levels of the above-discussed enzyme activities (i.e. endo-β-glucosidase Bl, tannase or anthocyanase) or which are capable of producing a novel mutant form of the enzymes responsible for these activities having an enhanced property (such as, for example, a more desired pH, or temperature optima, or greater tolerance to ethanol or sugar inhibition, etc.). As used herein, a "mutant" form of an enzyme (such as, As used herein, a "mutant" form of an enzyme (such as, for example, endo-β-glucosidase B1) is intended to include enzymes which are naturally produced, and which have been produced as the result of the mutagenesis of either and *Aspergillus niger* Bl cell, or a cell which contains a nucleic acid that encodes an enzyme of *Aspergillus niger* Bl strain CMI CC 324626. The present invention is intended to include both mutants of *Aspergillus niger* Bl strain CMI CC 324626, and mutant forms of the *Aspergillus niger* Bl strain CMI CC 324626 enzymes which are the subject of the present invention.

The present invention is also directed toward the "functional derivatives" of any of the above-described enzymes. A "functional derivative" of an enzyme (such as, for example, endo-β-glucosidase B1) is a compound which possesses a biological activity (either functional or structural) that is substantially similar to a biological activity of the enzyme. The term "functional derivative" is intended to include those "fragments," or "variants" of the enzyme which have an enzymatic activity that is substantially similar to that of the natural enzyme. A "fragment" of a molecule such as endo-β-glucosidase B1, is meant to refer to any polypeptide subset of the molecule. A "variant" of a molecule such as endo-β-glucosidase B1 is meant to refer to a molecule substantially similar in structure and function to either the entire molecule, or to a fragment thereof and which has been produced synthetically, or chemically (i.e. not through the mutation of the gene sequences which encode the enzyme). A molecule is said to be "substantially similar" to another molecule if both molecules have substantially similar structures or if both molecules possess a similar biological activity. Thus, provided that two molecules possess a similar activity, they are considered variants as that term is used herein even if the structure of one of the molecules is not found in the other, or if the sequence of amino acid residues is not identical.

### THE ENDO-BETA-GLUCOSIDASE B1, AND ITS FUNCTIONAL DERIVATIVES, AND MUTANTS

The novel endo-beta-glucosidase of the present invention is referred to herein as "endo-beta-glucosidase Bl." The present invention relates to the production, isolation and utilisation of this enzyme, substantially free of natural contaminants, as well as the functional derivatives of this enzyme. An "endo-beta-glucosidase" is an enzyme capable of hydrolyzing (i.e. cleaving) a glucosyl or glycosyl bond of a glycoside. A "glycoside" is an acetal of a sugar (a "glycone") and an alcohol (an "aglycone"). Of particular concern to the present invention are glycosides in which the aglycone is a monoterpene or an odorous fused oil (such as, for example, benzyl alcohol, or 2-phenyl ethanol). Monoterpenes, and their synthesis are reviewed by Schuette, H.R. *(Prog. Botan.* **46**:119-139 (1984)), and Akhila, A. *et. al. (Indian Perfum.* **27**:174-196 (1983)); all of which references are hereby incorporated by reference. Glucosyl derivatives of monoterpenes are reviewed by Stahl-Biskup, E. *(Flav. Fragr. J.* **2**:85-82 (1987), which reference is herein incorporated by reference). A "glucosyl bond", as used herein, is intended to refer to the bond between a monoterpene or other agylcone and a sugar residue. The glucosyl bond which is the preferred substrate of the enzymes of the present invention is an "O-glucosyl bond". Cleavage of such a bond therefore results in the release of a free monotorpene or other aglycone. The presence of such a glucosyl bond is a characteristic of a "glucosyl derivative." Examples of glucosyl derivatives of monoterpenes include O-alpha-L-rhamnopyranosyl-beta-D-glucopyranosides, and O-alpha-L-arabinofuranosyl-beta-D-glycopyranosides. The enzyme can also cleave a glycosyl bond, which is the characteristic bond of a "glycosyl derivative"; thus, the enzyme has "glycosidase activity".

As discussed above, flavorful and aromatic "free" monoterpenes are naturally converted into non-flavorful and non-odorous glucosyl and glycosyl derivatives ("bound monoterpenes"), and vice versa. Examples of the extent of this conversion are shown in Table 1. Table 1 shows the relative amounts of free monoterpenes and "bound" (i.e. glycosylated) monoterpene derivatives in various plant products. The effect of this conversion is to enhance the concentration of the monoterpene or other aglycone. As used herein, the term "enhance" is intended to indicate that the characteristic under discussion (i.e. concentration, activity, etc.) is greater under the "enhancing" conditions (i.e. high temperature, ethanol content, sugar concentration, etc.) than under opposite conditions (i.e. low temperature, no ethanol, no sugar, etc.).

**TABLE 1**

| RELATIVE AMOUNTS OF FREE AND BOUND MONOTERPENES IN A FEW FRUIT PRODUCTS | | | |
|---|---|---|---|
| VARIETY | µg LINALOOL PER LITER | | |
| | FREE | BOUND | RATIO (FREE/BOUND) |
| Jonathan Apple Juice | | | |
| Fresh | 553 | 922 | 1.67 |
| 1/4 dilution of concentrate | 1826 | 1660 | 0.91 |
| Shoumouti Orange Juice | | | |
| Fresh | 1839 | 4243 | 2.31 |
| 1/4 dilution of concentrate | 4661 | 11531 | 2.47 |
| White Grapefruit | 4066 | 11808 | 2.90 |
| Lisbon Variety Lemon Juice | 14010 | 45664 | 3.26 |
| Muscat of Alexandria Juice | 3539 | 13650 | 3.86 |

The endo-beta-glucosidase enzyme produced by A. niger B1 has a molecular weight of about 120,000 daltons by gel filtration, however, non-denaturing gradient gel electrophoresis showed the enzyme to have a molecular weight of 168,000 daltons, and to be composed of 4 identical subunits of 42,000 daltons each. The enzyme has an isoelectric point of about pI = 3.9 as determined by isoelectric focusing on a polyacrylamide gel. The enzyme exhibits a pronounced activity in media containing ethanol, and the activity is enhanced up to about 50% ethanol. The enzyme has a Kₘ of 1.0 mM for PNPG (p-nitrophenyl β-D-glucopyranoside and a Km of 1.25 mM for geranyl-β-rutonoside substrates. The enzyme is extremely stable, especially to heat. It is active from very low temperatures (i.e. about 10°C) to very high temperatures (i.e. temperatures above 60°C). The optimum temperature for enzyme activity is approximately 65°C. The endo-β-glucosidase B1 enzyme is extracellular and has an optimum activity at a pH in the range of about pH 3.4. The rather low pH at which this β-glucosidase is active and the thermal stability as well as its functioning at high concentrations of ethanol are unique and highly useful in the treatment of wines. The novel glucosidase of the present invention additionally exhibit a "cellobiase" activity, and thus is capable of use in the production of fuel from cellulose.

The concentrations of free and bound monoterpenes are preferably determined according to the method of Dimitriadis et. al. (Amer J. Enol. Vitic. 35:66-71 (1984) which reference is herein incorporated by reference). The activity of the endo-β-glucosidase of the present invention is preferably determined by standard methods using either PNPG or geranyl beta rutinoside as substrates.

The endo-β-glucosidase B1 enzyme and its functional derivatives can be effectively used to cleave the glucosyl bonds of monoterpene glycosides of a large variety of plants. The endo-β-glucosidase B1 enzyme of the present invention and its functional derivatives can, therefore, be used to increase the flavor of aroma of plant-derived foods, etc., by hydrolyzing the glucosyl bonds of glucosyl or glycosyl derivatives of "flavor-important monoterpenes" which are naturally present in plant materials. Examples of such "flavor-important monoterpenes include the monoterpenes of fruit (such as, for example, apples, passion fruit, oranges, grapes, grapefruit, lemon, etc.), and fruit juices (such as non-fermented fruit juices, beer, wine, liqueurs, liquors, etc.). Also included within the present invention are the "flavor-important monoterpenes" of spices (such as ginger, mint, cinnamon, pepper, etc.), tea, coffee, cocoa, etc. The enzymes of the present invention are also capable of hydrolyzing the glucosyl and glycosyl derivatives of the "fragrance-important" monoterpenes of fragrant plants (such as roses, geraniums, and other flowering plants). The endo-β-glucosidase B1 enzyme of the present invention and its functional derivatives are also capable of hydrolyzing the glucosyl and glycosyl derivatives of the monoterpenes of tobacco and similar plants. These enzymes are additionally capable of hydrolyzing the glucosyl and glycosyl derivatives of the monoterpenes of grains, vegetables, and similar plants.

Since the hydrolysis of glucosyl or glycosyl derivatives of monoterpenes of any of the above plants results in the release of additional "free" monoterpenes, and since such free monoterpenes are the compounds which impart the characteristic flavor, aroma or fragrance to such plant materials, the endo-β-glucosidase B1 enzyme of the present invention and its functional derivatives may be employed to enhance the flavor, aroma, or fragrance of such plant materials. Hence, these enzymes can be used to produce more flavorful foods, and beverages, as well as more fragrant spices, perfumes, etc. Flavor-important and fragrance-important monoterpenes are reviewed by Rapp, A. et. al. (Experientia 42:873-884 (1986)); Strauss, C. R. et. al. (ACS Symp. Ser. (Biogener. Aromas) 317:222-242 (1986)); Williams, P.J. et al. (In: Topics Flav. Res. (Proc. Intl. Conf.), Berger, R. et al. (eds.), Eichhorn, H., Marling-Hangenham, FRG, pages 335-352 (1985)); Kogami, K. (Koryo 145:11-21 (1985)); all of which references are hereby incorporated by reference.

As discussed below, certain plants (such as cassava (Manihot escolentum)) contain toxic glucosides. The endo-β-glucosidase B1 enzyme of the present invention and its functional derivatives can be used to detoxify the edible plant parts, and to increase its flavor.

The endo-β-glucosidase B1 enzyme of the present invention and its functional derivatives can also be used to isolate flavors and essences from plant materials. For example, by incubating passion fruit juice with the enzymes of the present invention it is possible to increase benzaldehyde ("almond essence") content.

### THE TANNASE AND ANTHOCYANASE ACTIVITIES OF A. NIGER B1

Mycelia of A. niger B1 (preferably immobilized in Ca alginate) were found to have an anthocyanase and tannase activity when applied to isolated wine glucosides. These activities can be used for the production of blush wines, juices, etc., in the food industry.

Since these activities have low molecular weights, they may be purified from an extract of A. niger by applying such an extract to a molecular weight sizing column (or by subjecting it to gel filtration, molecular sieve dialysis, electrophoresis, etc.). Fractions exhibiting tannase or anthocyanase activity can then be purified by standard assay methods, known to those of ordinary skill.

In the production of wine, the grape skin is an important source of flavor and aroma conferring compounds. Thus it is highly desirable to increase the amount of contact time which the skins have with the wine. Unfortunately, the grape skins also contain (and release into the must or the wine)tannins and anthocyanins which can impart an objectionable flavor or aroma to the wine. The tannase and anthocyanase activities of A. niger B1 are capable of hydrolyzing the undesired, excess tannins and anthocyanins which result from such prolonged skin contact. Thus, their use permits wine producers to increase skin contact time without impairing the flavor or aroma of the wine.

Although, as discussed above, it is often desirable to provide a tannase or anthocyanase activity to a wine (especially a "blush" wine), such activities are not desired in the case of a finished red wine. The tannase and anthocyanase activities of A. niger B1 can be readily removed from a sample or extract which contains them by virtue of their low molecular weight by molecular weight sieving techniques.

### PRODUCTION OF THE ENZYMES OF THE PRESENT INVENTION

When the above-described A. niger B1 strain, or equivalent is grown on a suitable medium, such as that used in the process for obtaining the desired strain (described above), or on a medium similar to such medium that but using rutin as sole carbon source, the desired enzymes can be obtained.

Although the preferred method for preparing the novel enzymes of the present invention is by the fermentation of *Aspergillus niger* Bl strain CMI CC 324626, it is to be understood that the present invention also includes the preparation of the enzyme by synthetic chemical, as well as by recombinant DNA, techniques. Thus, the present invention also includes nucleic acid molecules, such as RNA and DNA (and especially cDNA) which encode the amino acid sequences of the enzymes of the present invention, and mutants and derivatives of these enzymes.

The gene or cDNA sequence which encodes the "desired" enzyme of the present invention (i.e. endo-β-glucosidase Bl, obtainable from *Aspergillus niger* Bl) strain CMI CC 324626, or functional equivalents thereof) can be isolated by any of a variety of means. A preferred method for isolating nucleic acid sequences which encode the desired enzyme involves the screening of a genomic DNA or cDNA library with one or more oligonucleotide probes that encode a fragment for the desired enzyme.

In order to produce the oligonucleotide probes referred to above, the amino acid sequence of purified desired enzyme is determined. This may be accomplished through the use of any of the several methods known to those of ordinary skill. Although it is possible to determine the entire amino acid sequence of the protein, it is preferable to determine the sequence of peptide fragments of the protein. The desired enzyme is fragmented as with cyanogen bromide, or with proteases such as papain, chymotrypsin, or trypsin (Oike, Y., et al., J. Biol. Chem. 257:9751-9758 (1982); Liu, C., et al., Int. J. Pept. Protein Res. 21:209-215 (1983)). The resulting peptides are separated by reverse-phase HPLC and subjected to amino acid sequencing, preferably by automated sequenators.

Once one or more suitable peptide fragments have been sequenced, the DNA sequences capable of encoding them are examined. Because the genetic code is degenerate, more than one codon may be used to encode a particular amino acid (Watson, J.D., In: Molecular Biology of the Gene, 3rd Ed., W.A. Benjamin, Inc., Menlo Park, CA (1977), pp. 356-357). The peptide fragments are analyzed to identify sequences of amino acids which may be encoded by oligonucleotides having the lowest degree of degeneracy. This is preferably accomplished by identifying sequences that contain amino acids which are encoded by only a single codon.

Although occasionally an amino acid sequence may be encoded by only a single oligonucleotide, frequently the amino acid sequence may be encoded by any of a set of similar oligonucleotides. Importantly, whereas all the members of this set contain oligonucleotides which are capable of encoding the peptide fragment and, thus, potentially contain the same oligonucleotide sequence as the gene which encodes the peptide fragment, only one member of the set contains the nucleotide sequence that is identical to the nucleotide sequence of the actual encoding sequence. Because this member is present within the set, and is capable of hybridizing to DNA even in the presence of the other members of the set, it is possible to employ the unfractionated set of oligonucleotides in the same manner in which one would employ a single oligonucleotide to clone the actual encoding sequence that encodes the peptide.

Using the genetic code (Watson, J.D., In: Molecular Biology of the Gene, 3rd Ed., W.A. Benjamin, Inc., Menlo Park, CA (1977)), one or more different oligonucleotides can be identified, each of which would be capable of encoding the peptides of the desired enzyme. The probability that a particular oligonucleotide will, in fact, constitute the actual desired enzyme encoding sequence can be estimated by considering abnormal base pairing relationships and the frequency with which a particular codon is actually used (to encode a particular amino acid) in eukaryotic cells. Such "codon usage rules" are disclosed by Lathe, R., et al., J. Molec. Biol. 183:1-12 (1985). Using the "codon usage rules" of Lathe, a single oligonucleotide, or a set of oligonucleotides, that contains a theoretical "most probable" nucleotide sequence capable of encoding the desired enzyme peptide sequences is identified.

The oligonucleotide, or set of oligonucleotides, containing the theoretical "most probable" sequence capable of encoding the desired enzyme peptide fragment is used to identify the sequence of a complementary oligonucleotide or set of oligonucleotides which is capable of hybridizing to the "most probable" sequence, or set of sequences. An oligonucleotide containing such a complementary sequence can be employed as a probe to identify and isolate the desired enzyme gene (or cDNA) using, for example the techniques disclosed by Maniatis, T., et al. (In: Molecular Cloning A Laboratory Manual, Cold Spring Harbor Press, Cold Spring Harbor, NY (1982)).

Thus, in summary, the actual identification of the peptide sequences of the desired enzyme permits the identification of a theoretical "most probable" DNA sequence, or a set of such sequences, capable of encoding such a peptide. By constructing an oligonucleotide complementary to this theoretical sequence (or by constructing a set of oligonucleotides complementary to the set of "most probable" oligonucleotides), one obtains a DNA molecule (or set of DNA molecules), capable of functioning as a probe to identify and isolate the desired enzyme encoding nucleic acid sequences.

A suitable oligonucleotide, or set of oligonucleotides, which is capable of encoding a fragment of the desired enzyme (or which is complementary to such an oligonucleotide, or set of oligonucleotides) is identified using the above-described procedure, synthesized, and hybridized by means well known in the art, against a DNA or, more preferably, a cDNA preparation derived from cells which are capable of expressing the desired enzyme. The source of DNA or cDNA used will preferably have been enriched for sequences which encode the desired enzyme. Such enrichment can most easily be obtained from cDNA obtained by extracting RNA from cells which produce high levels of the desired enzyme. Techniques of nucleic acid hybridization are disclosed by Maniatis, T., et al. (In: Molecular Cloning, A Laboratory Manual, Cold Spring Harbor Laboratories, Cold Spring Harbor, NY (1982)), and by Haymes, B.D., et al. (In: Nucleic Acid Hybridization, A Practical Approach, IRL Press, Washington, DC (1985)), which references are herein incorporated by reference.

Genomic DNA can be extracted and purified from suitable cells by means well known in the art. Genomic DNA may or may not include naturally occurring introns. Moreover, such genomic DNA may be obtained in association with the 5' promoter region of the desired enzyme gene sequences. To the extent that a host cell can recognize the transcriptional regulatory and translational initiation signals associated with the expression of the protein, then the region 5' may be retained and employed for transcriptional and translational initiation regulation.

Alternatively, mRNA can be isolated from Aspergillus niger strain B1, and used to produce cDNA by means well known in the art. The cDNA may be cloned and the resulting clone screened with an appropriate probe for cDNA coding for the desired sequences. Once the desired clone has been isolated, the cDNA may be manipulated in substantially the same manner as the genomic DNA. However, with cDNA there will be no introns or intervening sequences. For this reason, a cDNA molecule which encodes the desired enzyme is the preferred genetic sequence of the present invention.

Suitable DNA preparations are enzymatically cleaved, or randomly sheared, and ligated into recombinant vectors to form a gene library. Such vectors can then be screened with one or more oligonucleotide probes in order to identify a sequence which encodes the desired enzyme.

To facilitate the detection of the desired enzyme encoding sequence, the above-described DNA probe may be labeled with a detectable group. Such detectable group can be any material having a detectable physical or chemical property. Such materials have been well-developed in the field of immunoassays and in general most any label useful in such methods can be applied to the present invention. Particularly useful are enzymatically active groups, such as enzymes (see Clin. Chem. 22:1243 (1976)), enzyme substrates (see British Pat. Spec. 1,548,741), coenzymes (see U.S. Pat. Nos. 4,230,797 and 4,238,565) and enzyme inhibitors (see U.S. Pat. No. 4,134,792); fluorescers (see Clin. Chem. 25:353 (1979)); chromophores; luminescers such as chemiluminescers and bioluminescers (see Clin. Chem. 25:512 (1979)); specifically bindable ligands; proximal interacting pairs; and radioisotopes such as ³H, ³⁵S, ³²p, ¹²⁵I and ¹⁴C. Such labels and labeling pairs are detected on the basis of their own physical properties (e.g., fluorescers, chromophores and radioisotopes) or their reactive or binding properties (e.g., enzymes, substrates, coenzymes and inhibitors). For example, a cofactor-labeled probe can be detected by adding the enzyme for which the label is a cofactor and a substrate for the enzyme. For example, one can use an enzyme which acts upon a substrate to generate a product with a measurable physical property. Examples of the latter include, but are not limited to, beta-galactosidase, alkaline phosphatase and peroxidase.

General procedures for hybridization are disclosed, for example, in Maniatis, T., Molecular Cloning A Laboratory Manual, Cold Spring Harbor Press, Cold Spring Harbor, NY (1982) and in Haymes, B.T., et al., Nucleic Acid Hybridization a Practical Approach, IRL Press, Oxford, England (1985). Those members of the above-described gene library which are found to be capable of such hybridization are then analyzed to determine the extent and nature of the desired enzyme encoding sequences which they contain.

Techniques such as, or similar to, those described above have successfully enabled the cloning of genes for human aldehyde dehydro-genases (Hsu, L.C. et al., Proc. Natl. Acad. Sci. USA 82:3771-3775 (1985)), fibronectin (Suzuki, S. et al., Eur. Mol. Biol. Organ. J. 4:2519-2524 (1985)), the human estrogen receptor gene (Walter, P. et al., Proc. Natl. Acad. Sci. USA 82:7889-7893 (1985)), tissue-type plasminogen activator (Pennica, D. et al., Nature 301:214-221 (1983)) and human term placental alkaline phosphatase complementary DNA (Kam, W. et al., Proc. Natl. Acad. Sci. USA 82:8715-8719 (1985)).

### RECOMBINANT DNA EXPRESSION OF THE ENZYMES OF THE PRESENT INVENTION

The desired enzyme encoding sequences, obtained through the methods described above, may be operably linked to an expression vector, and introduced into prokaryotic or eukaryotic cells in order to produce the desired enzyme or its functional derivatives.

A DNA sequence encoding the desired enzyme or its functional derivatives may be recombined with vector DNA in accordance with conventional techniques, including blunt-ended or staggered-ended termini for ligation, restriction enzyme digestion to provide appropriate termini, filling in of cohesive ends as appropriate, alkaline phosphatase treatment to avoid undesirable joining, and ligation with appropriate ligases. Techniques for such manipulations are disclosed by Maniatis, T., et al., supra, and are well known in the art.

A nucleic acid molecule, such as DNA, is said to "capable of expressing" a polypeptide if it contains nucleotide sequences which contain transcriptional and translational regulatory information and such sequences are "operably linked" to nucleotide sequences which encode the polypeptide. An operable linkage is a linkage in which the regulatory DNA sequences and the DNA sequence sought to be expressed are connected in such a way as to permit gene expression. The precise nature of the regulatory regions needed for gene expression may vary from organism to organism, but shall in general include a promoter region which, in prokaryotes, contains both the promoter (which directs the initiation of RNA transcription) as well as the DNA sequences which, when transcribed into RNA, will signal the initiation of protein synthesis. Such regions will normally include those 5'-non-coding sequences involved with initiation of transcription and translation, such as the TATA box, capping sequence, CAAT sequence, and the like.

If desired, the non-coding region 3' to the gene sequence coding for the protein may be obtained by the above-described methods. This region may be retained for its transcriptional termination regulatory sequences, such as termination and polyadenylation. Thus, by retaining the 3'-region naturally contiguous to the DNA sequence coding for the protein, the transcriptional termination signals may be provided. Where the transcriptional termination signals are not satisfactorily functional in the expression host cell, then a 3' region functional in the host cell may be substituted.

Two DNA sequences (such as a promoter region sequence and a desired enzyme encoding sequence) are said to be operably linked if the nature of the linkage between the two DNA sequences does not (1) result in the introduction of a frame-shift mutation, (2) interfere with the ability of the promoter region sequence to direct the transcription of the desired enzyme encoding gene sequence, or (3) interfere with the ability of the desired enzyme gene sequence to be transcribed by the promoter region sequence. Thus, a promoter region would be operably linked to a DNA sequence if the promoter were capable of effecting transcription of that DNA sequence.

Thus, to express the protein, transcriptional and translational signals recognized by an appropriate host are necessary.

The present invention encompasses the expression of the desired enzyme (or a functional derivative thereof) in either prokaryotic or eukaryotic cells. Preferred prokaryotic hosts include bacteria such as E. coli, Bacillus, Streptomyces, Pseudomonas, Salmonella, Serratia, etc. The most preferred prokaryotic host is E. coli. Bacterial hosts of particular interest include E. coli K12 strain 294 (ATCC 31446), E. coli X1776 (ATCC 31537), E. coli W3110 (F⁻, lambda⁻, prototrophic (ATCC 27325)), and other enterobacterium such as Salmonella typhimurium or Serratia marcescens, and various Pseudomonas species. The procaryotic host must be compatible with the replicon and control sequences in the expression plasmid.

To express the desired enzyme (or a functional derivative thereof) in a prokaryotic cell (such as, for example, E. coli, B. subtilis, Pseudomonas, Streptomyces, etc.), it is necessary to operably link the desired enzyme encoding sequence to a functional prokaryotic promoter. Such promoters may be either constitutive or, more preferably, regulatable (i.e., inducible or derepressible). Examples of constitutive promoters include the int promoter of bacteriophage λ, the bla promoter of the β-lactamase gene of pBR322, and the CAT promoter of the chloramphenicol acetyl transferase gene of pPR325, etc. Examples of inducible prokaryotic promoters include the major right and left promoters of bacteriophage λ (P_{L} and P_{R}), the trp, recA, lacZ, lacI, and gal promoters of E. coli, the α-amylase (Ulmanen, I., et. al., J. Bacteriol. 162:176-182 (1985)) and the σ-28-specific promoters of B. subtilis (Gilman, M.Z., et al., Gene 32:11-20 (1984)), the promoters of the bacteriophages of Bacillus (Gryczan, T.J., In: The Molecular Biology of the Bacilli, Academic Press, Inc., NY (1982)), and Streptomyces promoters (Ward, J.M., et al., Mol. Gen. Genet. 203:468-478 (1986)). Prokaryotic promoters are reviewed by Glick, B.R., (J. Ind. Microbiol. 1:277-282 (1987)); Cenatiempo, Y. (Biochimie 68:505-516 (1986)); and Gottesman, S. (Ann. Rev. Genet. 18:415-442 (1984)).

Proper expression in a prokaryotic cell also requires the presence of a ribosome binding site upstream of the gene-encoding sequence. Such ribosome binding sites are disclosed, for example, by Gold, L., et al. (Ann. Rev. Microbiol. 35:365-404 (1981)).

Preferred eukaryotic hosts include yeast, fungi (especially Aspergillus), mammalian cells (such as, for example, human or primate cells) either in vivo, or in tissue culture. Mammalian cells provide post-translational modifications to protein molecules including correct folding or glycosylation at correct sites. Mammalian cells which may be useful as hosts include cells of fibroblast origin such as VERO or CHO-K1, and their derivatives.

For a mammalian host, several possible vector systems are available for the expression of the desired enzyme. A wide variety of transcriptional and translational regulatory sequences may be employed, depending upon the nature of the host. The transcriptional and translational regulatory signals may be derived from viral sources, such as adenovirus, bovine papilloma virus, Simian virus, or the like, where the regulatory signals are associated with a particular gene which has a high level of expression. Alternatively, promoters from mammalian expression products, such as actin, collagen, is preferable to ensure that the linkage between a eukaryotic promoter and a DNA sequence which encodes the desired enzyme (or a functional derivative thereof) does not contain any intervening codons which are capable of encoding a methionine (i.e., AUG). The presence of such codons results either in a formation of a fusion protein (if the AUG codon is in the same reading frame as the desired enzyme encoding DNA sequence) or a frame-shift mutation (if the AUG codon is not in the same reading frame as the desired enzyme encoding sequence).

The desired enzyme encoding sequence and an operably linked promoter may be introduced into a recipient prokaryotic or eukaryotic cell either as a non-replicating DNA (or RNA) molecule, which may either be a linear molecule or, more preferably, a closed covalent circular molecule. Since such molecules are incapable of autonomous replication, the expression of the desired enzyme may occur through the transient expression of the introduced sequence. Alternatively, permanent expression may occur through the integration of the intoduced sequence into the host chromosome.

In one embodiment, a vector is employed which is capable of integrating the desired gene sequences into the host cell chromosome. Cells which have stably integrated the introduced DNA into their chromosomes can be selected by also introducing one or more markers which allow for selection of host cells which contain the expression vector. The marker may provide for complement an auxotrophy in the host, biocide resistance, e.g., antibiotics, or heavy metals, such as copper, or the like. The selectable marker gene can either be directly linked to the DNA gene sequences to be expressed, or introduced into the same cell by co-transfection. Additional elements may also be needed for optimal synthesis of single chain binding protein mRNA. These elements may include splice signals, as well as transcription promoters, enhancers, and termination signals. cDNA expression vectors incorporating such elements include those described by Okayama, H., Mol. Cell. Biol. 3:280 (1983).

In a preferred embodiment, the introduced sequence will be incorporated into a plasmid or viral vector capable of autonomous replication in the recipient host. Any of a wide variety of vectors may be employed for this purpose. Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species. Preferred prokaryotic vectors include plasmids such as those capable of replication in E. coli (such as, for example, pBR322, ColE1, pSC101, pACYC 184, πVX. Such plasmids are, for example, disclosed by Maniatis, T., et al. (In: Molecular Cloning, A Laboratory Manual, Cold Spring Harbor Press, Cold Spring Harbor, NY (1982)). Bacillus plasmids include pC194, pC221, pT127, etc. Such plasmids are disclosed by Gryczan, T. (In: The Molecular Biology of the Bacilli, Academic Press, NY (1982), pp. 307-329). Suitable Streptomyces plasmids include pIJ101 (Kendall, K.J., et al., J. Bacteriol. 169:4177-4183 (1987)), and streptomyces bacteriophages such as φC31 (Chater, K.F., et al., In: Sixth International Symposium on Actinomycetales Biology, Akademiai Kaido, Budapest, Hungary (1986), pp. 45-54). Pseudomonas plasmids are reviewed by John, J. F., et al. (Rev. Infect. Dis. 8:693-704 (1986)), and Izaki, K. (Jpn. J. Bacteriol. 33:729-742 (1978)).

Preferred eukaryotic plasmids include BPV, vaccinia, SV40, 2-micron circle, etc., or their derivatives. Such plasmids are well known in the art (Botstein, D., et al., Miami Wntr. Symp. 19:265-274 (1982); Broach, J.R., In: The Molecular Biology of the Yeast Sac-charomyces: Life Cycle and Inheritance, Cold Spring Harbor Laboratory, Cold Spring Harbor, NY, p. 445-470 (1981); Broach, J.R., Cell 28:203-204 (1982); Bollon, D.P., et al., J. Clin. Hematol. Oncol. 10:39-48 (1980); Maniatis, T., In: Cell Biology: A Comprehensive Treatise, Vol. 3, Gene Expression, Academic Press, NY, pp. 563-608 (1980)).

Once the vector or DNA sequence containing the construct(s) has been prepared for expression, the DNA construct(s) may be introduced into an appropriate host cell by any of a variety of suitable means: transformation, transfection, conjugation, protoplast fusion, electroporation, calcium phosphate-precipitation, direct microinjection, etc. After the introduction of the vector, recipient cells are grown in a selective medium, which selects for the growth of vector-containing cells. Expression of the cloned gene sequence(s) results in the production of the desired enzyme, or its functional derivative.

The expressed protein may be isolated and purified in accordance with conventional conditions, such as extraction, precipitation, chromatography, affinity chromatography, electrophoresis, or the like.

In order to use the novel enzymes of the present invention to produce more flavorful foods, spices, beverages, etc., the enzyme is provided to a sample of plant material in "effective amount," and incubated in the presence of such material for a time sufficient to release a desired percentage of free monoterpenes from the initially present glucosyl or glycosyl derivatives which are naturally found in the plant. As used herein, an "effective amount" of the enzymes of the present invention is intended to refer to an amount of enzyme which is capable of effecting the release of an amount of free monoterpenes sufficient to alter the flavor, aroma, or fragrance of the substrate plant extract.

The enzyme may be provided directly to the plant (as by injection, spraying, etc.) or, more preferably, it may be applied to an extract of the plant (such as, for example, ground coffee, tobacco or tea leaves, fruit juice, pureed vegetables, wine, fruit juices etc.). Since some individuals may be allergic to the enzymes of the present invention, the most preferred means for treating such an extract with the enzyme comprises incubating the extract in the presence of fungal mycelia or, more preferably, and enzyme preparation, which has been immobilised onto an inert or insoluble carrier or matrix. In accordance with such a method, the plant extract is incubated in the presence of the mycelia of enzyme preparation by passing the extract through a column containing such immobilised mycelia or enzyme. The resulting treated extract can be recovered free of any of the novel enzymes of the present invention. Alternatively, the plant extract can be placed in contact with immobilized mycelia or enzyme, and, after treatment, the immobilized mycelia or enzyme can be removed from the sample by filtration, centrifugation etc. The immobilization of the fungal mycelia or novel enzymes of the present invention can be accomplished by any of a variety of means which are well known in the art. Alginates, Upergit C, of celluloses can be used to immobilize the mycelia. Cellulose is the preferred matrix for enzyme immobilization, and especially lignin-crosslinked cellulose such as that found in nut shells, etc. The crushed shells of hazelnuts comprise the most preferred cellulose matrix.

It is alternatively possible to produce a recombinant plant through the use of the techniques and methods of recombinant DNA technology. Such a plant may either be a higher plant (such as, for example, a fruit tree, a vegetable or tobacco plant, a grain or other plant used for food or beverage, a flowering plant, etc.) or a lower plant (such as yeast, mold, fungi, etc.). Such plants may, for example, be prepared by introducing a gene sequence capable of encoding one of the novel enzymes of the present invention into a desired plant cell. This may be accomplished by any suitable means. Such a plant would be capable of expressing the desired enzyme of the present invention, or one of its functional derivatives. The present invention, thus, also includes genetically engineered plants which contain and express the desired enzyme, or any of its functional derivatives, and whose fruit or flowers have enhanced flavor or fragrance due to the presence and expression of any of the novel enzymes of the present invention.

Of particular significance is the formation of a recombinant yeast cell (and especially a Brewer's yeast cell) which contains any of the novel enzymes of the present invention. Such a cell may advantageously be employed in the fermentation of beer, wine, etc. The fermentation products so produced will contain increased concentrations of monoterpenes, and will, therefore, have more flavor and aroma.

### Deposit in International Culture Collection

Aspergillus niger strain B1 was deposited with the Culture Collection of the Commonwealth Mycological Institute, Ferry Lane, Kew, Richmond, Surrey TW9 3AF, United Kingdom, under Deposit No. CMI CC 324626, on May 13, 1988, prior to the filing date of this application.

Having now generally described this invention, the same will be better understood by reference to certain specific examples which are included herein for purposes of illustration only and are not intended to be limiting of the invention, unless specified.

### EXAMPLE 1

### PRODUCTION OF ENDO-B-GLUCOSIDASE B1

A conventional A. niger strain Hebrew University, Department of Microbiology, Strain Collection of Faculty of Agriculture, Clone No. 5 was grown from spores on a medium containing the following: 0.4% purified monoterpene glycosides isolated and purified from Muscat of Alexandria wine (see below) as a sole carbon source; (NH)₂SO₄ 0.5 g/l, KH₂PO₄·3H₂O 0.2 g/l, MgSO₄ 0.2 g/l, CaCl₂·2H₂O 0.1 g/l, FeSO₄·6H₂O 0.001 g/l, ZnSO·₄H₂O 0.01/g/l, pH 3.4 adjusted by HCl. After incubation for several weeks in an agitating incubator at 30° at 100 rpm the slow growing medium was crushed by ultratourax and was further incubated for three days. The resulting massive mycelium formed, sporulated after several days, and served as a source for A. niger strain B1.

The production strain A. niger B1 was grown in a 10 liter fermentor with a medium similar to that described above, with rutin (Sigma Inc.) serving as a sole carbon source. Temperature was kept at 25°, agitation 200 rpm and aeration at 10 l/mil. After 48 hours the growing medium was filtered through cheesecloth, then through 0.45 micron membrane. The supernatant was ultrafiltered and concentrated to 20 ml with a 20,000 m.w. cut-off membrane. An activity of about 20 units was obtained.

### Monoterpene glycoside isolation and purification.

Thirty liters of vacuum dealcoholized wine (Muscat of Alexandria) were loaded on a C18 reversed phase absorbent (40-60 µ Merc Inc.) packed in a 60x2 cm flush chromatography glass column. The column was first flushed with water and the glycosides were then eluted by methanol. The methanol was evaporated and the remaining solution was lyophilized.

### Enzyme Assay

The β-glucosidase activity was assayed by incubating 25 µl of enzyme preparation in 1 ml of 1 mM PNPG (p-nitrophenyl-β-D-glucopyranoside) for 2 minutes at 35°. The reaction was terminated by adding 200 µl of 1M Na₂CO₃ and the OD was read at 399 nm.

### EXAMPLE 2

### QUANTITATION OF ENDO AND EXOCELLULAR B-GLUCOSIDASE B1 ACTIVITY

A. niger B1 was grown on two kinds of liquid medium containing either monoterpene glycosides (isolated from a Muscat of Alexandria wine according to Williams et al. (Phytochemistry 21:2013-2020 (1982))) or rutin 1 g/l. After 48 hours of incubation at 40 ml sample was filtered through a 0.45 µ nitrocellulose membrane to obtain 20 ml of filtrate, whereas the nonfiltered residue containing the mycelium was homogenized and centrifuged at 10,000 rpm for 20 minutes.

The supernatant and the filtered samples were then separately incubated with 20 ml of 1 mg/l of wine glycosides for 20 hours at 30°. Free monoterpenes were then analyzed according to Dimitriadis et al. (Amer J. Enol. Vitic. 35:66-71 (1984)). Characterization of the A. niger B1 β-glucosidase B1 activity included the determination of pH, ionic strength, temperature, and ethanol response curves, Kₘ, Vmax and Kᵢ for glucose, by standard procedures. In addition, the effect of various concentrations of grape juice (Muscat of Alexandria) sucrose, fructose and SO₂ on the enzyme activity was determined by conventional methods. These experiments revealed that glucose is a competitive inhibitor of the enzyme, and that the activity of the enzyme is enhanced by ethanol. The results of these experiments are shown in Tables 2 and 3.

**TABLE 2**

| **THE EFFECT OF MEDIUM CARBON SOURCE ACTIVITY ON EXTRA AND ENDOCELLULAR ENZYME ACTIVITY** | |
|---|---|
| ENZYME SOURCE | LINALOOL EQUIVALENT (mg/l) |
| Supernatant of N. niger B1 on monoterpene glycosides medium | 7.26 |
| Homogenate of A. niger B1 on monoterpene glycosides medium | 23.72 |
| Supernatant of A. niger B1 on a rutin medium | 13.28 |
| Homogenate of A. niger B1 on a rutin medium | 19.19 |

**TABLE 3**

| **PHYSICOCHEMICAL PROPERTIES OF ASPERGILLUS NIGER B1 ENDO-B-GLUCOSIDASE PREPARATION** | |
|---|---|
| PROPERTY | |
| Kₘ (PNPG) | 1.0 mM |
| Vmax (PNPG) | 0.22 µmol min⁻¹ mg⁻¹ prot |
| Kₘ (geranyl β rutinoside) | 1.25 mM |
| Vmax (geranyl β rutinoside) | 0.08 µmol min⁻¹ mg⁻¹ prot |
| Kᵢ (Glucose) | 40 mM |
| Temperature activity optimum | 65°C |
| Temperature stability half life at 60° | 2 hr |
| Activity after 7 days at 50° | 100% |
| Molecular weight | 120,000 |
| pH activity optimum | 3.4 |
| Isoelectric point | pI=3.9 |
| Activity between pH 1.5-5 | >50% |
| Activity at 12°C | >5% |
| Activity in the presence of 0-50% ethanol | >150% |
| Activity in the presence of 1M sucrose | 90% |
| Activity in the presence of 1M Fructose | 90% |
| Activity in the presence of 200ppm SO2 | 100% |

### EXAMPLE 3

### PURIFICATION OF ENDO-BETA-GLUCOSIDASE B1

### Isoelectric Focusing on Polyacrylamide Gel

Analytical isoelectric focusing was performed in 0.5 mm polyacrylamide gel (T=5%; C=3%) containing 2.4% weight per volume ampholine (pH=2.5-5) using a multiphor unit LKB 2117 at 2000 v 25W for 1 hour. The slab was sliced longitudinally into three strips. One strip was cross-sectioned into 0.5 cm-slices, which were dipped in 1 ml dd (double distilled) H₂O for several hours after which the pH gradient was established. The second strip was dipped in 100 mg/l x glue (5-Bromo-4-chloro-3-indolyl β-D-glucopyranoside) for several hours and the β-glucosidase B1 activity was visualized as previously described. The third strip was stained with a general protein stainer Coumassie brilliant blue 250-R.

### Gel Filtration Chromatography

Gel filtration was performed using Sepharose CL-6B column and a MWFG 1000 molecular weight marker kit (Sigma Tech. Bull. GF-3).

### EXAMPLE 4

### ENHANCEMENT OF FLAVOR BY ENDO-BETA-GLUCOSIDASE B1

Purified endo-β-glucosidase B1 was immobilized on Upergite C and contacted with Muscat wine for 24 hours at 16°C. There resulted a greatly enhanced aroma. A chemical assay was carried out and it was found that the quantity of the free monoterpenes was about doubled, including tertiary monoterpene alcohols. These were separated by liquid-liquid freon extraction, separated by gas chromatography and identified by mass spectrometry. Sensory evaluation by the official testing panel of the Israeli Wine Institute using the duo trio method confirmed these results statistically.

### EXAMPLE 5

### TANNASE AND ANTHOCYANASE ACTIVITIES OF A. NIGER B1

Aspergillus niger strain B1 was immobilized in calcium alginate and contacted with red wine anthocyanin for 24 hours at 30°C. The solution was bleached.

The crude endo-β-glucosidase B1 was found to have a pronounced effect on wines and other plant material and was found to be capable or reducing the anthocyanin and tannin content of wine. The enzyme may therefore be employed to produce "blush" (i.e. rose or paler red wines) from ordinary (i.e. red) wine, or enable a long period of grape skin contact prior to or during fermentation, resulting in increased aroma extraction from the skin and low tannin and anthocyanin content due to enzymatic degradation.

### EXAMPLE 6

### ENHANCEMENT OF MONOTERPENE CONCENTRATION BY ENDO-BETA-GLUCOSIDASE B1

Eight different wines were incubated in the presence of an effective amount of the endo-β-glucosidase B1 of the present invention in order to determine the effectiveness and specificity of the enzyme. Treatment was for 24 hours at room temperature (25-30°C). The results of this experiment are shown in Figure 1. Before incubation ("FREE" in Figure 1), and after the conclusion of treatment ("BOUND" in Figure 1), the amount and chemical nature of free monoterpenes were determined. The following monoterpenes were detected: (1) cis-furanlinalooloxide; (2) trans-furanlinalooloxide; (3) linalool; (4) hotrienol; (5) alpha-terpineol; (6) cis-pyranlinalooloxide; (7) trans-pyranlinalooloxide; (8) citronellol; (9) nerol; (10) geraniol; (11) benzyl alcohol; and (12) 2-phenyl ethanol. The bracketed numbers correspond to the numbers in Figure 1. This experiment showed that the endo-β-glucosidase B1 of the present invention can be used to increase the concentration of flavor-important monoterpenes in wine. The ability to increase the concentration of the flavor-important monoterpene, linalool, demonstrates that the endo-β-glucosidase B1 of the present invention can be used to hydrolyze glucosyl bonds between sugars and "tertiary monoterpene alcohols" (i.e. monoterpenes which are tertiary alcohols).

As indicated in Figure 1, a novel endo-beta-glucosidase of the present invention is not only capable of increasing the concentration of the flavour and fragrance important monoterpenes in wine, but is also capable of increasing the concentration of odorous fused oils (such as benzyl alcohol and 2-phenyl ethanol, etc.) in wine. Thus, this Example demonstrates that the enzyme is able to hydrolyze glycosides containing aglycones other than monoterpenes.

### EXAMPLE 7

### ENHANCEMENT OF FRAGRANCE BY ENDO-BETA-GLUCOSIDASE B1

In a manner similar to that described above, the novel enzyme of the present invention, was incubated with monoterpene glycosides present in a variety of fragrant plant materials (such as rose petals, geranium, lemon grass, mint leaves, etc.) and found to be able to hydrolyze these compounds, and thereby increase the fragrance or aroma of extracts of these plant materials. Thus, the enzymes of the present invention can be used to enhance the fragrance or aroma of perfumes, essential oils, essences, etc.

### EXAMPLE 8

### DETOXIFICATION OF CYANOGENIC COMPOUNDS BY ENDO-BETA-GLUCOSIDASE B1

Certain plants (such as cassava, almond, etc.) contain toxic glycosides (such as cyanogenic glucosides). Such plants must therefore be treated before they will be suitable as foodstuffs. The novel enzymes of the present invention possess beta glucosidase activity, and thus have the ability to cleave such glycosidic bonds (in the same manner as glucosyl bonds can be cleaved by the enzymes). Thus, by incubating such toxic plants (or products of such plants) in the presence of the enzymes of the present invention, it is possible to detoxify such toxic plants and thereby increase their value as a foodstuff. It is alternatively possible to produce a recombinant cassava plant which naturally expresses any of the enzymes of the present invention.

## Claims (Claims for the following Contracting State(s): AT, BE, CH, DE, FR, GB, GR, IT, LI, LU, NL, SE)

1. A flavour- or fragrance-enhancing enzyme obtainable from Aspergillus niger B1 which is strain CMI CC 324626, or a functional derivative of said enzyme, characterised in that the enzyme has endo-beta glucosidase activity, has an optimum of glucosidase activity at about pH 3.4, has enhanced glucosidase activity in a culture medium that has up to about 50% alcohol content and has glucosidase activity at 60°C.

2. Aspergillus niger B1 strain CMI CC 324626 or a mutant thereof which has an enzyme according to claim 1.

3. A method of hydrolyzing a glycoside present in a fruit or plant extract or fermentation product thereby to produce an aglycone, the method comprising:
providing to the plant extract or the fermentation product an effective amount of an enzyme or a functional derivative thereof according to claim 1
incubating the extract or the product, with the enzyme under conditions sufficient to permit the enzyme or a functional derivative thereof to hydrolyse the glycoside.

4. A method as claimed in claim 3 wherein the aglycone is a monoterpene or an odorous fused oil such that the presence of the aglycone causes the plant extract or fermentation product to have enhanced flavour or fragrance.

5. A method as claimed in claim 3 or 4 wherein the enzyme is provided to the extract or product by contacting the extract with the enzyme or functional derivative thereof in a form substantially free of natural contaminants.

6. A method as claimed in claim 5 wherein the enzyme or functional derivative thereof is immobilised on an insoluble carrier.

7. A method as claimed in claim 3 or 4 wherein the enzyme or functional derivative thereof is provided to the extract or product by contacting the extract with mycelia of an Aspergillus niger B1 strain CMI CC 324626 or a mutant thereof according to claim 2.

8. A method as claimed in claim 7 wherein the mycelia are immobilised on an insoluble carrier.

9. A method for detoxifying a plant extract which contains a cyanogenic glycoside which comprises providing to the plant extract an effective amount of an enzyme or functional derivative thereof as defined in any of claims 1, 3 or 4.

10. A method of hydrolyzing a glycoside present in a plant thereby to produce an aglycone, the method comprising:
providing to the plant an effective amount of an enzyme or functional derivative thereof according to claim 1; and
cultivating the plant under conditions sufficient to permit the enzyme or functional derivative thereof to hydrolyze the glycoside.

11. A method as claimed in claim 10 wherein the aglycone is a monoterpene, or an odorous fused oil such that the presence of the aglycone causes the plant to have enhanced flavour or fragrance.

12. A process of obtaining a strain of Aspergillus niger or a mutant thereof according to claim 2, the process comprising growing Aspergillus niger in a medium where one or more monoterpene glycosides is/are the sole carbon source and selecting a strain having an enzyme or functional derivative thereof as defined in claim 1.

13. A process of obtaining an enzyme or functional derivative thereof as defined in claim 1, the process comprising isolating said enzyme or functional derivative thereof from an Aspergillus niger strain CMI CC 324626 or from a mutant thereof obtained according to the method of claim 12.

## Claims (Claims for the following Contracting State(s): ES)

1. A process for the preparation of a flavour- or fragrance-enhancing enzyme or functional derivative thereof which comprises obtaining the enzyme or functional derivative thereof from Aspergillus niger B1 strain CMI CC 324626 or a mutant thereof, wherein the enzyme or functional derivative thereof has an optimum of glucosidase activity at about pH 3.4 has enhanced glucosidase activity in a culture medium that has up to about 50% alcohol content and has glucosidase activity at 60°C.

2. Aspergillus niger B1 strain CMI CC 324626, or a mutant thereof which has an enzyme as described in claim 1.

3. A process for the production of a recombinant DNA molecule which encodes an enzyme or functional derivative thereof as described in claim 1, the process comprising coupling together successive nucleotides and/or ligating oligo- and/or polynucleotides.

4. A process as claimed in claim 3 wherein the recombinant DNA molecule is additionally capable of expressing the enzyme or functional derivative thereof.

5. A method of hydrolyzing a glycoside present in a fruit or plant extract or fermentation product thereby to produce an aglycone, the method comprising:
providing to the plant extract or the fermentation product an effective amount of an enzyme, which enzyme is endo-beta-glucosidase B1 or a functional derivative thereof preparable by a process according to claim 1; and
incubating the extract or the product, with the enzyme under conditions sufficient to permit the enzyme to hydrolyse the glycoside.

6. A method as claimed in claim 5 wherein the aglycone is a monoterpene or an odorous fused oil and wherein the presence of the aglycone causes the plant extract or fermentation product to have enhanced flavour or fragrance.

7. A method as claimed in claim 5 or 6 wherein the enzyme is provided to the extract or product by contacting the extract with the enzyme in a form substantially free of natural contaminants.

8. A method as claimed in claim 7 wherein the enzyme is immobilised on an insoluble carrier.

9. A method as claimed in claim 5 or 6 wherein the enzyme is provided to the extract or product by contacting the extract with mycelia of Aspergillus niger strain B1 or a mutant thereof, according to claim 2.

10. A method as claimed in claim 9 wherein the mycelia are immobilised on an insoluble carrier.

11. A method for detoxifying a plant extract which contains a cyanogenic glycoside which comprises providing to the plant extract an effective amount of an enzyme or functional derivative thereof preparable by a process according to claim 1 which enzyme or functional derivative thereof has an endo-beta-glucosidase activity.

12. A method of hydrolyzing a glycoside present in a plant thereby to produce an aglycone, the method comprising:
providing to the plant an effective amount of an enzyme which is endo-beta-glucosidase B1 or functional derivative thereof preparable by a process according to claim 1; and
cultivating the plant under conditions sufficient to permit the enzyme or functional derivative thereof to hydrolyze the glycoside.

13. A method as claimed in claim 12 wherein the aglycone is a monoterpene, or an odorous fused oil, and wherein the presence of the aglycone causes the plant to have enhanced flavour or fragrance.

14. A process of obtaining a strain of Aspergillus niger strain CMI CC 324626 or a mutant thereof as described in claim 2, the process comprising growing Aspergillus niger in a medium where one or more monoterpene glycoside is the sole carbon source.

15. A process of obtaining an enzyme from Aspergillus niger strain CMI CC 324626 or from a mutant thereof as described in claim 2, the process comprising culturing cells of Asp. niger as prepared according to claim 14, and isolating the enzyme; the enzyme being the enzyme or the functional derivative thereof described in claim 1.

## Patentansprüche (Patentansprüche für folgende(n) Vertragsstaat(en): AT, BE, CH, DE, FR, GB, GR, IT, LI, LU, NL, SE)

1. Ein geschmacks- und geruchsverstärkendes Enzym, welches aus Aspergillus niger B1, der der Stamm CMI CC 324626 ist, oder ein funktionelles Derivat dieses Enzyms, dadurch gekennzeichnet, daß das Enzym endo-beta-Glukosidase-Aktivität, ein Optimum an Glukosidase-Aktivität bei etwa pH 3,4, eine erhöhte Glukosidase-Aktivität in einem etwa 50% Alkohol enthaltenden Kulturmedium und Glukosidase-Aktivität bei 60°C besitzt.

2. Aspergillus niger B1 Stamm CMI CC 324626 oder eine Mutante desselben, welche ein Enzym gemäß Anspruch 1 aufweist.

3. Verfahren zur Hydrolysierung eines Glykosids, welches in einem Frucht- oder Pflanzenextrakt oder einem Fermentationsprodukt enthalten ist, um dadurch ein Aglykon zu erzeugen, welches Verfahren umfaßt:
Bereitstellen einer wirksamen Menge eines Enzyms oder eines funktionellen Derivates desselben gemäß Anspruch 1 an einen Pflanzenextrakt oder an das Fermentationsprodukt
Inkubieren des Extraktes oder des Produktes mit dem Enzym unter Bedingungen, die ausreichen, um dem Enzym oder dem funktionellen Derivat desselben die Hydrolyse des Glykosids zu ermöglichen.

4. Verfahren nach Anspruch 3, bei welchem das Aglykon ein Monoterpen oder ein riechendes Fuselöl ist, so daß die Gegenwart des Aglykons den Pflanzenextrakt oder das Fermentationsprodukt veranlaßt, einen verstärkten Geschmack oder Geruch zu besitzen.

5. Verfahren nach Anspruch 3 oder 4, bei welchem das Enzym an den Extrakt oder das Produkt durch Kontaktieren des Extraktes mit dem Enzym oder dessen funktionellem Derivat in im wesentlichen von natürlichen Verunreinigungen freier Form bereitgestellt wird.

6. Verfahren nach Anspruch 5, bei welchem das Enzym oder dessen funktionelles Derivat an einen unlöslichen Träger immobilisiert ist.

7. Verfahren nach Anspruch 3 oder 4, bei welchen das Enzym oder dessen funktionelles Derivat an den Extrakt oder das Produkt durch Kontaktieren des Extraktes mit Mycel von Aspergillus niger B1 Stamm CMI CC 324626 oder von einer Mutante davon gemäß Anspruch 2 bereitgestellt wird.

8. Verfahren nach Anspruch 7, bei welchem das Mycel an einem unlöslichen Träger immobilisiert ist.

9. Verfahren zur Entgiftung eines Pfanzenextraktes, welcher ein Blausäureglykosid enthält, welches Verfahren das Bereitstellen einer wirksamen Menge eines Enzyms oder eines funktionellen Derivates davon wie in den Ansprüchen 1, 3 oder 4 definiert an den Pflanzenextrakt umfaßt.

10. Verfahren zur Hydrolysierung eines in einer Pflanze enthaltenen Glykosids um dadurch ein Aglykon zu erzeugen, welches Verfahren umfaßt:
Bereitstellen einer wirksamen Menge eines Enzyms oder funktionellen Derivates davon gemäß Anspruch 1 an die Pflanze; und
Kultivierung der Pflanze unter Bedingungen, die ausreichen, um dem Enzym oder funktionellen Derivat desselben die Hydrolyse des Glykosids zu erlauben.

11. Verfahren nach Anspruch 10, bei welchem das Aglykon ein Monoterpen oder ein riechendes Fuselöl ist, so daß die Gegenwart des Aglykons die Pflanze veranlaßt einen verstärkten Geschmack oder Geruch zu besitzen.

12. Verfahren zur Gewinnung eines Stammes von Aspergillus niger oder einer Mutante desselben gemäß Anspruch 2, wobei das Verfahren das Züchten von Aspergillus niger in einem Medium, in welchem ein oder mehrere monoterperne Glykoside die einzige Kohlenstoffquelle sind, und die Auswahl eines Stammes umfaßt, welcher ein Enzym oder ein funktionelles Derivat desselben wie in Anspruch 1 definiert enthält.

13. Verfahren zur Gewinnung eines Enzyms oder funktionellen Derivates desselben gemäß Anspruch 1, wobei das Verfahren das Isolieren des genannten Enzyms oder funktionellen Derivates desselben aus einem Aspergillus niger Stamm CMI CC 324626 oder einer Mutante desselben, welcher/welche gemäß dem Verfahren nach Anspruch 12 erhalten wurde, umfaßt.

## Patentansprüche (Patentansprüche für folgende(n) Vertragsstaat(en): ES)

1. Verfahren zur Herstellung eines geschmacks- oder geruchsverstärkenden Enzyms oder eines funktionellen Derivats desselben, welches Verfahren die Gewinnung des Enzyms oder funktionellen Derivats desselben aus Aspergillus niger B1 Stamm CMI CC 324626 oder einer Mutante davon umfaßt, wobei das Enzym oder funktionelle Derivat desselben ein Optimum an Glukosidasaktivität bei ungefähr pH 3,4, erhöhte Glukosidasaktivität in einem bis zu 50% Alkohol enthaltenden Kulturmedium und Glukosidaseaktivität bei 60°C besitzt.

2. Aspergillus niger B1 Stamm CMI CC 324626, oder eine Mutante desselben, welcher/welche ein Enzym wie in Anspruch 1 beschrieben aufweist.

3. Verfahren zur Herstellung eines rekombinanten DNA-Moleküls, welches für ein Enzym oder ein funktionelles Derivat desselben gemäß Anspruch 1 kodiert, wobei das Verfahren das Zusammenkoppeln aufeinanderfolgender Nukleotide und/oder Ligieren von Oligo- und/oder Polynukleotiden umfaßt.

4. Verfahren nach Anspruch 3, bei welchem das rekombinante DNA-Molekül zusätzlich in der Lage ist, Enzyme oder funktionelle Derivate desselben zu exprimieren.

5. Verfahren zur Hydrolyse eines Glykosids, welches in einem Frucht- oder Pflanzenextrakt oder einem Fermentationsprodukt enthalten ist, um dadurch ein Aglykon zu erzeugen, welches Verfahren umfaßt:
Bereitstellen einer wirksamen Menge eines Enzyms, welches Enzym endo-beta-Gukosidase B1 oder ein funktionelles Derivat desselben ist, das gemäß Anspruch 1 herstellbar ist, an einen Pflanzenextrakt oder an das Fermentationsprodukt; und
Inkubieren des Extraktes oder des Produktes mit dem Enzym unter Bedingungen, die ausreichen, um dem Enzym die Hydrolyse des Glykosids zu ermöglichen.

6. Verfahren nach Anspruch 5, bei welchem das Aglykon ein Monoterpen oder ein riechendes Fuselöl ist und bei welchem die Gegenwart des Aglykons den Pflanzenextrakt oder das Fermentationsprodukt veranlaßt, einen verstärkten Geschmack oder Geruch zu besitzen.

7. Verfahren nach Anspruch 5 oder 6, bei welchem das Enzym an den Extrakt oder das Produkt durch Kontaktieren des Extraktes mit dem Enzym in im wesentlichen von natürlichen Verunreinigungen freier Form bereitgestellt wird.

8. Verfahren nach Anspruch 7, bei welchem das Enzym an einem unlöslichen Träger immobilisiert ist.

9. Verfahren nach Anspruch 5 oder 6, bei welchen das Enzym an den Extrakt oder das Produkt durch Kontaktieren des Extraktes mit Mycel von Aspergillus niger B1 Stamm CMI CC 324626 oder von einer Mutante davon gemäß Anspruch 2 bereitgestellt wird.

10. Verfahren nach Anspruch 9, bei welchem das Mycel an einem unlöslichen Träger immobilisiert ist.

11. Verfahren zur Entgiftung eines Pfanzenextraktes, welcher ein Blausäureglykosid enthält, welches Verfahren das Bereitstellen einer wirksamen Menge eines Enzyms oder eines funktionellen Derivates davon, welches nach dem Verfahren gemäß Anspruch 1 herstellbar ist, wobei das Enzym oder das funktionelle Derivat desselben endo-beta-Glukosidase-Aktivität aufweist.

12. Verfahren zur Hydrolysierung eines in einer Pflanze enthaltenen Glykosids um dadurch ein Aglykon zu erzeugen, welches Verfahren umfaßt:
Bereitstellen einer wirksamen Menge eines Enzyms, welches endo-beta-Glukosidase B1 oder ein funktionelles Derivat davon ist, welches nach einem Verfahren gemäß Anspruch 1 herstellbar ist, an die Pflanze; und
Kultivierung der Pflanze unter Bedingungen, die ausreichen, um dem Enzym oder funktionellen Derivat desselben die Hydrolyse des Glykosids zu erlauben.

13. Verfahren nach Anspruch 12, bei welchem das Aglykon ein Monoterpen oder ein riechendes Fuselöl ist, und wobei die Gegenwart des Aglykons die Pflanze veranlaßt einen verstärkten Geschmack oder Geruch zu besitzen.

14. Verfahren zur Gewinnung eines Stammes von Aspergillus niger CMI CC 324626 oder einer Mutante desselben wie in Anspruch 2 beschrieben, wobei das Verfahren das Züchten von Aspergillus niger in einem Medium, in welchem ein oder mehrere monoterperne Glykoside die einzige Kohlenstoffquelle sind, umfaßt.

15. Verfahren zur Gewinnung eines Enzyms aus Aspergillus niger Stamm CMI CC 324626 oder einer Mutante desselben wie in Anspruch 2 beschrieben, wobei das Verfahren das Züchten von nach dem Verfahren gemäß Anspruch 14 erzeugten Asp. niger Zellen und das Isolieren des Enzyms umfaßt; wobei das Enzym jenes Enzym oder funktionelles Derivat desseben ist, welches in Anspruch 1 beschrieben ist.

## Revendications (Revendications pour l'(les) Etat(s) contractant(s) suivant(s): AT, BE, CH, DE, FR, GB, GR, IT, LI, NU, NL, SE)

1. Enzyme exhausteur de goût ou d'odeur, pouvant être obtenu à partir de la souche CMI CC 324626 d'Aspergillus Niger B1, ou dérivé fonctionnel de cet enzyme, caractérisé en ce que l'enzyme possède une activité endobêta-glucosidase, possède une activité glucosidase optimale à un pH d'environ 3,4, possède une activité glucosidase augmentée dans un milieu de culture contenant jusqu'environ 50% d'alcool, et possède une activité glucosidase à 60°C.

2. Souche CMI CC 324626 d'Aspergillus Niger B1, ou un mutant de celle-ci, ayant un enzyme suivant la revendication 1.

3. Procédé d'hydrolyse d'un glucoside présent dans un extrait de fruit ou de plante ou dans un produit de fermentation, de manière à produire une aglycone, caractérisé en ce qu'il comprend :
l'addition à l'extrait de plante ou au produit de fermentation d'une quantité efficace d'un enzyme ou d'un dérivé fonctionnel de celui-ci suivant la revendication 1; et
l'incubation de l'extrait ou du produit, avec l'enzyme, dans des conditions suffisantes pour permettre à l'enzyme ou à son dérivé fonctionnel d'hydrolyser le glucoside.

4. Procédé suivant la revendication 3, caractérisé en ce que l'aglycone est un monoterpène ou une huile fondue odorante, de telle sorte que la présence de l'aglycone confère à l'extrait de plante ou au produit de fermentation un goût ou une odeur augmentés.

5. Procédé suivant l'une quelconque des revendications 3 ou 4, caractérisé en ce que l'enzyme est ajouté à l'extrait ou au produit par mise en contact de l'extrait et de l'enzyme, ou son dérivé fonctionnel, sous une forme essentiellement libre de contaminateurs naturels.

6. Procédé suivant la revendication 5, caractérisé en ce que l'enzyme ou son dérivé fonctionnel est immobilisé sur un support insoluble.

7. Procédé suivant l'une quelconque des revendications 3 ou 4, caractérisé en ce que l'enzyme ou son dérivé fonctionnel est ajouté à l'extrait ou au produit par mise en contact de l'extrait et de mycélium de la souche CMI CC 324626 d'Aspergillus Niger B1, ou d'un mutant de celle-ci, suivant la revendication 2.

8. Procédé suivant la revendication 7, caractérisé en ce que le mycélium est immobilisé sur un support insoluble.

9. Procédé de détoxication d'un extrait de plante contenant un glucoside cyanogène, caractérisé en ce qu'il comprend l'addition à l'extrait de plante d'une quantité efficace d'un enzyme, ou d'un dérivé fonctionnel de celui-ci, comme défini dans l'une quelconque des revendications 1, 3 ou 4.

10. Procédé d'hydrolyse d'un glucoside présent dans une plante, de manière à produire une aglycone, caractérisé en ce qu'il comprend :
l'addition à la plante d'une quantité efficace d'un enzyme ou d'un dérivé fonctionnel de celui-ci suivant la revendication 1; et
la culture de la plante dans des conditions suffisantes pour permettre à l'enzyme ou à son dérivé fonctionnel d'hydrolyser le glucoside.

11. Procédé suivant la revendication 10, caractérisé en ce que l'aglycone est un monoterpène, ou une huile fondue odorante, de telle sorte que la présence de l'aglycone confère à la plante un goût ou une odeur augmentés.

12. Procédé pour obtenir une souche d'Aspergillus Niger ou un mutant de celle-ci suivant la revendication 2, caractérisé en ce qu'il comprend le développement d'Aspergillus Niger dans un milieu où un ou plusieurs glucosides monoterpènes est/sont la seule source de carbone, et la sélection d'une souche ayant un enzyme ou un dérivé fonctionnel de celui-ci comme défini dans la revendication 1.

13. Procédé pour obtenir un enzyme ou un dérivé fonctionnel de celui-ci comme défini dans la revendication 1, caractérisé en ce qu'il comprend l'isolement de cet enzyme ou de son dérivé fonctionnel d'une souche CMI CC 324626 d'Aspergillus Niger, ou d'un mutant de celle-ci, obtenue suivant le procédé suivant la revendication 12.

## Revendications (Revendications pour l'(les) Etat(s) contractant(s) suivant(s): ES)

1. Procédé de préparation d'un enzyme exhausteur de goût ou d'odeur, ou d'un dérivé fonctionnel de cet enzyme, caractérisé en ce qu'il comprend l'obtention de l'enzyme ou de son dérivé fonctionnel à partir de la souche CMI CC 324626 d'Aspergillus Niger B1, ou d'un mutant de celle-ci, et en ce que l'enzyme ou son dérivé fonctionnel possède une activité glucosidase optimale à un pH d'environ 3,4, possède une activité glucosidase augmentée dans un milieu de culture contenant jusqu'environ 50% d'alcool, et possède une activité glucosidase à 60°C.

2. Souche CMI CC 324626 d'Aspergillus Niger B1, ou un mutant de celle-ci, ayant un enzyme suivant la revendication 1.

3. Procédé de production d'une molécule d'ADN recombinant qui code pour un enzyme ou un dérivé fonctionnel de celui-ci comme décrit dans la revendication 1, caractérisé en ce qu'il comprend le couplage de nucléotides successifs et/ou la ligation d'oligo- et/ou de polynucléotides.

4. Procédé suivant la revendication 3, caractérisé en ce que la molécule d'ADN recombinant est en outre capable d'exprimer l'enzyme ou son dérivé fonctionnel.

5. Procédé d'hydrolyse d'un glucoside présent dans un extrait de fruit ou de plante ou dans un produit de fermentation de manière à produire une aglycone, caractérisé en ce qu'il comprend :
l'addition à l'extrait de plante ou au produit de fermentation d'une quantité efficace d'un enzyme, qui est l'endo-bêta-glucosidase B1 ou un dérivé fonctionnel de celle-ci, préparable selon un procédé suivant la revendication 1; et
l'incubation de l'extrait ou du produit, avec l'enzyme, dans des conditions suffisantes pour permettre à l'enzyme d'hydrolyser le glucoside.

6. Procédé suivant la revendication 5, caractérisé en ce que l'aglycone est un monoterpène ou une huile fondue odorante, et en ce que la présence de l'aglycone confère à l'extrait de plante ou au produit de fermentation un goût ou une odeur augmentés.

7. Procédé suivant l'une quelconque des revendications 5 ou 6, caractérisé en ce que l'enzyme est ajouté à l'extrait ou au produit par mise en contact de l'extrait et de l'enzyme sous une forme essentiellement libre de contaminateurs naturels.

8. Procédé suivant la revendication 7, caractérisé en ce que l'enzyme ou son dérivé fonctionnel est immobilisé sur un support insoluble.

9. Procédé suivant l'une quelconque des revendications 5 ou 6, caractérisé en ce que l'enzyme est ajouté à l'extrait ou au produit par mise en contact de l'extrait et de mycélium de la souche Aspergillus Niger B1, ou d'un mutant de celle-ci, suivant la revendication 2.

10. Procédé suivant la revendication 9, caractérisé en ce que le mycélium est immobilisé sur un support insoluble.

11. Procédé de détoxication d'un extrait de plante contenant un glucoside cyanogène, caractérisé en ce qu'il comprend l'addition à l'extrait de plante d'une quantité efficace d'un enzyme, ou d'un dérivé fonctionnel de celui-ci, préparable par un procédé suivant la revendication 1, l'enzyme ou son dérivé fonctionnel ayant une activité endo-bêta-glucosidase.

12. Procédé d'hydrolyse d'un glucoside présent dans une plante de manière à produire une aglycone, caractérisé en ce que le procédé comprend :
l'addition à la plante d'une quantité efficace d'un enzyme, qui est l'endo-bêta-glucosidase B1 ou un dérivé fonctionnel de celle-ci, préparable par un procédé suivant la revendication 1; et
la culture de la plante dans des conditions suffisantes pour permettre à l'enzyme ou à son dérivé fonctionnel d'hydrolyser le glucoside.

13. Procédé suivant la revendication 12, caractérisé en ce que l'aglycone est un monoterpène, ou une huile fondue odorante, et en ce que la présence de l'aglycone confère à la plante un goût ou une odeur augmentés.

14. Procédé pour obtenir une souche d'Aspergillus Niger CMI CC 324626, ou un mutant de celle-ci, suivant la revendication 2, caractérisé en ce qu'il comprend le développement d'Aspergillus Niger dans un milieu où un ou plusieurs glucosides monoterpènes sont la seule source de carbone.

15. Procédé pour obtenir un enzyme à partir de la souche CMI CC 324626 d'Aspergillus Niger, ou d'un mutant de celle-ci, comme décrit dans la revendication 2, caractérisé en ce qu'il comprend la culture de cellules d'Aspergillus Niger préparées suivant la revendication 14, et l'isolement de l'enzyme; l'enzyme étant l'enzyme, ou son dérivé fonctionnel, comme défini dans la revendication 1.
